# FASCICULE DE BREVET EUROPEEN

(11) **EP 1 858 760 B1**
(45) Date de publication et mention de la délivrance du brevet: **17.09.2008**
(21) Numéro de dépôt: 06726068.7
(22) Date de dépôt: 10.03.2006
(51) Int. Cl.: B65B 55/10, A61L 2/26

(54) **CAISSE POUR PRODUITS STERILES**
KASTEN FÜR STERILE PRODUKTE
BOX FOR STERILE PRODUCTS

(30) Priorité: 10.03.2005 FR 0550635
(43) Date de publication de la demande: 28.11.2007
(73) Titulaire: DS Smith Kaysersberg, 68320 Kunheim (FR)
(72) Inventeur: MAILLOT, Guillaume, 68000 Colmar (FR)
(74) Mandataire: David, Daniel
(86) Numéro de dépôt international: PCT/FR2006/000537
(87) Numéro de publication internationale: WO 2006/095097

(56) Documents cités:
- US-A- 4 550 546
- US-A- 4 603 538
- US-A- 5 518 115

## Description

La présente invention concerne une caisse réalisée par le pliage et la mise en volume d'une plaque en matériau semi rigide plastique et destinée à recevoir des produits stériles.

Pour l'emballage et l'expédition de produits stériles, tels que des instruments ou articles médicaux ou chirurgicaux, par exemple des boîtes ou des sachets contenant des seringues, on utilise des caisses en matière plastique alvéolaire que l'on traite ensemble dans une enceinte de stérilisation avec les produits qu'elles contiennent. Une méthode consiste à remplir la caisse avec le produit concerné venu de fabrication et non encore stérile, puis à soumettre l'ensemble à un traitement de stérilisation par un gaz approprié, tel que l'EtO (oxyde d'éthylène) dans une enceinte hermétique. Après un temps d'exposition déterminé, jusqu'à 75 heures, le gaz est évacué de l'enceinte et remplacé par de l'air avant que l'on sorte le produit de l'enceinte. Le produit traité est ainsi expédié dans sa caisse sans autre manipulation avant son arrivée à destination.

Le gaz utilisé pour le traitement de stérilisation étant par nature très toxique puisqu'il doit supprimer tout germe, on s'assure que des traces de gaz ne restent pas emprisonnées à l'intérieur de l'emballage. Les procédures de traitement mises en oeuvre sont élaborées pour assurer une sécurité totale à l'égard des opérateurs et agents chargés de manipuler les caisses notamment en sortie de l'enceinte de traitement.

On emploie volontiers, pour confectionner les caisses d'emballage, des plaques de matériau plastique alvéolaire. Ce matériau est à la fois léger et résistant. Il se compare au carton ondulé, et présente l'avantage supplémentaire d'être durable et plus résistant à la contamination que ce dernier.

On désigne par l'expression plaques en matériau plastique alvéolaire des plaques constituées d'au moins deux feuilles planes parallèles maintenues à distance l'une de l'autre par des cloisons parallèles entre elles. Les feuilles et les cloisons ménagent entre elles des canaux longitudinaux que l'on désigne alvéoles dans le domaine. Ces plaques sont obtenues par extrusion d'une matière plastique choisie en fonction de l'application, telle qu'une polyoléfine, à travers une filière rectiligne. Immédiatement en aval de la tête d'extrusion la matière encore à l'état plastique passe entre des plaques de calibrage pour figer la forme de la plaque.

Ce type de plaque est maintenant d'usage courant et est susceptible de se substituer au carton ondulé pour certaines applications comme celle rapportée ci-dessus. La caisse courante est la caisse américaine. Elle comprend une ceinture à quatre panneaux prolongés chacun de part et d'autre par des rabats que l'on replie à angle droit pour former le fond et le haut ou couvercle de la caisse.

Dans le cas d'une caisse utilisée pour les traitements de stérilisation, on perfore les feuilles de couverture constituant la plaque alvéolaire d'une multitude de trous pour assurer une circulation du gaz à travers celle-ci pendant la phase de traitement dans l'enceinte et éviter que celui-ci s'accumule et forme des poches résiduelles en fin de traitement lors de la phase d'élimination du gaz.

La demanderesse s'est fixé l'objectif d'améliorer la circulation des gaz à travers la caisse quand les rabats sont pliés à angle droit et la caisse refermée prête à être expédiée directement après son traitement dans l'enceinte de stérilisation gazeuse.

La caisse, conforme à l'invention, pour produits stériles constituée de panneaux adjacents formant une ceinture et de rabats prolongeant les panneaux et formant les parois de fond et de dessus perpendiculaires à la ceinture, la ceinture et les rabats étant découpés dans une plaque en matériau plastique alvéolaire comportant au moins deux feuilles de couverture maintenues à distance l'une de l'autre par une pluralité d'entretoises ménageant des alvéoles longitudinales parallèles entre elles, est caractérisée par le fait qu'au moins l'une des feuilles de couverture étant perforée pour permettre le passage d'un gaz de stérilisation au travers de ladite feuille de couverture, les perforations permettant le passage du gaz mais empêchant le passage des poussières, des découpes larges traversent au moins l'autre feuille de couverture de la plaque pour assurer la communication desdites alvéoles avec l'extérieur.

Cette solution procure une grande sécurité quant à la circulation des gaz et surtout en ce qui concerne l'élimination après traitement. En effet les alvéoles forment des cheminées débouchant directement à l'extérieur grâce à ces découpes. Ces découpes sont suffisamment larges pour chevaucher au moins la largeur d'une alvéole. De préférence les perforations sont ménagées sur la feuille de la plaque à l'intérieur de la caisse et les découpes sur la feuille extérieure.

Conformément à une caractéristique préférée, les découpes sont ménagées le long de lignes d'articulation des rabats sur la ceinture.

On décrit ci-après plus en détail un mode de réalisation de l'invention, en référence aux dessins annexés sur lesquels
- la figure 1 représente une plaque de matière plastique alvéolaire ;
- la figure 2 montre la structure de la plaque par une coupe selon la direction 2-2 de la figure 1 ;
- la figure 3 montre une caisse formée par mise en volume de la plaque de la figure 1, les rabats étant repliés.

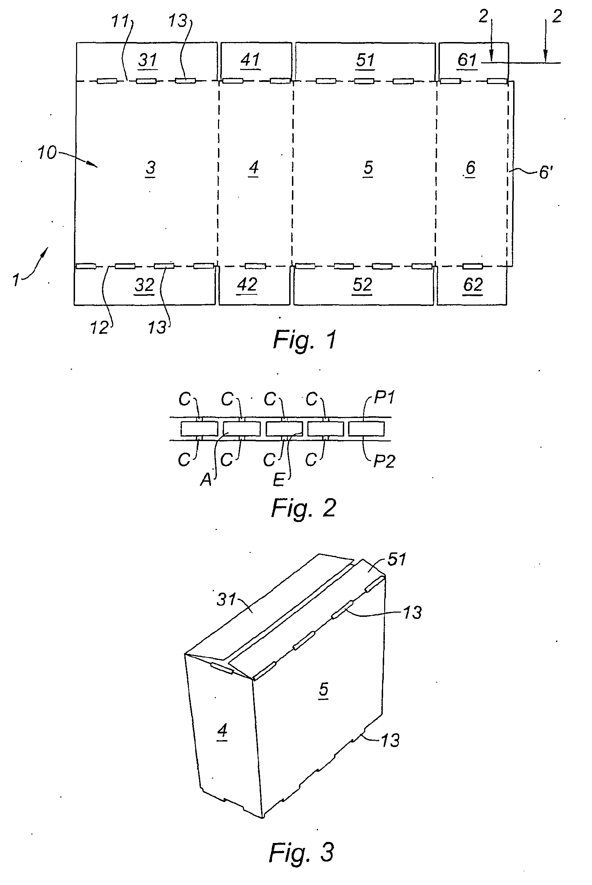

Comme on le voit sur la figure 1, une plaque 1 est classiquement découpée de manière à définir quatre panneaux adjacents 3, 4, 5 et 6, de la ceinture 10. Le panneau 6 comprend un tenon latéral 6'. Chacun des panneaux se prolonge de chaque côté par un rabat 31 et 32, 41 et 42, 51 et 52, 61 et 62 respectivement. Une ligne d'articulation 11 et 12 délimite la séparation entre la ceinture et les rabats. L'articulation est formée par écrasement de la plaque le long de cette ligne. Au lieu d'une seule plaque repliée sur elle-même, la ceinture peut être formée à partir de deux moitiés assemblées le long de deux soudures.

On voit sur la figure 2 la plaque 1, en coupe selon la direction 2 de la figure 1, agrandie. On distingue deux parois de couverture P1 et P2 maintenues à distance l'une de l'autre par des entretoises E espacées les unes des autres et parallèles entre elles. Les entretoises et les feuilles de couverture définissent des canaux ou alvéoles A parallèles entre eux. La direction des alvéoles est perpendiculaire par rapport aux lignes d'articulation 11 et 12. Elle pourrait former un autre angle que droit mais elle n'est pas parallèle à celles-ci.

Dans la solution de l'art antérieur, afin de permettre la circulation des gaz à travers la plaque, des trous calibrés, C, sont pratiqués dans la plaque. Leur diamètre est suffisant pour rendre la plaque perméable aux gaz ou aux vapeurs. Cependant il n'est pas trop important pour éviter que les salissures ne la traversent aussi.

La réalisation de ces trous n'est pas simple car il faut s'assurer que l'ensemble des alvéoles soit perméable pour éviter précisément tout risque d'accumulation de gaz toxique. Dans un processus industriel, cette contrainte entraîne des coûts que l'on a cherché à éviter.

Conformément à l'invention, on a résolu le problème de la mise en communication de chacune des alvéoles avec l'extérieur en pratiquant des découpes transversales à la direction des alvéoles le long des lignes de pliage 11 et 12. Ces découpes 13 sont pratiquées avec un couteau au travers d'au moins l'une des feuilles de couverture, la feuille de couverture extérieure dans ce cas, ou à travers les deux feuilles de couverture. La longueur de chaque découpe et le nombre des découpes le long de la plaque sont déterminés expérimentalement. Ces découpes peuvent être de simples entailles pratiquées avec une lame à travers une ou les deux feuilles de couverture. Elles peuvent aussi être pratiquées de manière à ce que les bords découpés soient espacés l'un de l'autre. L'espacement peut être de quelques millimètres.

On a représenté sur la figure 3, la caisse formée après pliage et mise en volume de la plaque de la figure 1. Par le pliage des rabats, les bords des découpes 13 sont dégagés et les alvéoles forment des cheminées débouchant à l'air libre. On observe que les découpes sont pratiquées de manière à ce que de préférence toutes les alvéoles communiquent avec l'extérieur de la caisse. Avantageusement on a alterné les découpes sur les lignes de pliage des rabats du fond et du haut.

Si nécessaire, on peut pratiquer des découpes sur les rabats du fond et du haut de manière que les alvéoles débouchent des deux côtés.

Selon un autre mode de réalisation non représenté, on découpe des lamelles dans la feuille externe des panneaux de la caisse. Ces lamelles ont une faible largeur et s'étendent en travers de plusieurs alvéoles.

## Revendications

1. Caisse pour produits stériles constituée de panneaux formant une ceinture (10) et de rabats prolongeant les panneaux et formant les parois de fond et de dessus perpendiculaires à la ceinture, la ceinture et les rabats étant découpés dans une plaque en matériau plastique alvéolaire comportant au moins deux feuilles (P1, P2) de couverture maintenues à distance l'une de l'autre par une pluralité d'entretoises (E) ménageant des alvéoles longitudinales parallèles entre elles, **caractérisée par le fait qu'**au moins l'une des feuilles de couverture étant perforée (C) pour permettre le passage d'un gaz de stérilisation au travers de ladite feuille de couverture, les perforations permettant le passage du gaz mais empêchant le passage des poussières, des découpes (13) larges traversent au moins l'autre feuille de couverture de la plaque pour assurer la communication desdites alvéoles avec l'extérieur.

2. Caisse selon la revendication précédente dont les perforations (C) sont ménagées sur la feuille (P1) de la plaque à l'intérieur de la caisse, et les découpes (13) sur la feuille (P2) extérieure.

3. Caisse selon la revendication 1 ou 2 dont les découpes sont ménagées le long des lignes d'articulation des rabats (31 32 41 42 51 52 61 62) sur la ceinture.

4. Caisse selon la revendication précédente, dont les découpes (13) le long de la ligne d'articulation des rabats de fond alternent avec les découpes (13) le long de la ligne d'articulation des rabats de la paroi du haut.

5. Caisse selon la revendication 3 ou 4 dont une partie des alvéoles (A) débouche sur des découpes (13) à leur deux extrémités.

6. Caisse selon l'une des revendications 1 à 5 dont une seule des feuilles de couverture est perforée.

## Claims

1. Box for sterile products, comprising panels which form a belt (10) and flaps which extend the panels and form the base and top walls perpendicular to the belt, the belt and the flaps being cut out of a sheet made of alveolar plastic material comprising at least two cover leaves (P1, P2) which are maintained at a spacing one from the other by a plurality of spacers (E) providing parallel longitudinal alveoli between them, **characterised in that** at least one of the cover leaves being perforated (C) in order to allow passage of a sterilisation gas through said cover leaf, the perforations allowing passage of the gas but preventing passage of dust, wide cuts (13) penetrate at least the other cover leaf of the sheet in order to ensure communication of said alveoli with the exterior.

2. Box according to the preceding claim, the perforations (C) of which are provided on the leaf (P1) of the sheet in the interior of the box, and the cuts (13) on the exterior leaf (P2).

3. Box according to claim 1 or 2, the cuts of which are provided along lines of articulation of the flaps (31 32 41 42 51 52 61 62) on the belt.

4. Box according to the preceding claim, the cuts (13) of which along the line of articulation of the base flaps alternate with the cuts (13) along the line of articulation of the flaps of the top wall.

5. Box according to claim 3 or 4, one part of the alveoli (A) of which opens out into cuts (13) at the two ends thereof.

6. Box according to one of the claims 1 to 5, only one of the cover leaves of which is perforated.

## Patentansprüche

1. Kasten für sterile Produkte, bestehend aus eine Zarge (10) bildenden Tafeln und die Tafeln verlängernden Klappen, die die zur Zarge senkrechten Boden- und Deckelwände bilden, wobei die Zarge und die Klappen aus einem Kunststoffmaterial mit zellenartiger Struktur ausgeschnitten sind, die mindestens zwei Deckfolien (P1, P2) umfasst, die durch eine Mehrzahl von Stegen (E), die zueinander parallele Längszellen bilden, in einem Abstand voneinander gehalten werden, **dadurch gekennzeichnet, dass** mindestens eine der Deckfolien perforiert ist (C), um den Durchgang eines Sterilisierungsgases durch die Deckfolie zu gestatten, wobei die Perforierungen den Durchgang des Gases gestatten, aber den Durchgang von Staub verhindern, und breite Ausschnitte (13) mindestens die andere Deckfolie der Platte durchqueren, um die Verbindung der Zellen mit dem Äußeren zu gewährleisten.

2. Kasten nach dem vorhergehenden Anspruch, dessen Perforierungen (C) auf der Folie (P1) der Platte im Inneren des Kastens und dessen Ausschnitte (13) auf der äußeren Folie (P2) vorgesehen sind.

3. Kasten nach Anspruch 1 oder 2, dessen Ausschnitte längs Gelenklinien der Klappen (31, 32, 41, 42, 51, 52, 61, 62) auf der Zarge vorgesehen sind.

4. Kasten nach dem vorhergehenden Anspruch, dessen Ausschnitte (13) längs der Gelenklinie der Bodenklappen mit den Ausschnitten (13) längs der Gelenklinie der Klappen der Deckelwand abwechseln.

5. Kasten nach Anspruch 3 oder 4, bei dem ein Teil der Zellen (A) an ihren beiden Enden in die Ausschnitte (13) ausmündet.

6. Kasten nach einem der Ansprüche 1 bis 5, bei dem nur eine der Deckfolien perforiert ist.
